# EUROPEAN PATENT SPECIFICATION

(11) **EP 1 427 394 B1**
(45) Date of publication and mention of the grant of the patent: **14.03.2007**
(21) Application number: 02759994.3
(22) Date of filing: 13.09.2002
(51) Int. Cl.: A61K 9/127, A61K 47/48, A61K 31/66, A61P 17/02

(54) **PROCESS FOR ACCELERATING RECOVERY FROM TRAUMA BY USING APOPTOSIS-MIMICKING SYNTHETIC OR NATURAL ENTITIES**
VERWENDUNG VON SYNTHETISCHEN ODER NATÜRLICHEN APOPTOSE-VORTÄUSCHENDEN EIINHEITEN ZUR BESCHLEUNIGTEN ERHOLUNG VON VERLETZUNGEN
PROCEDE PERMETTANT D'ACCELERER LA GUERISON D'UN TRAUMATISME AU MOYEN D'ENTITES SYNTHETIQUES OU NATURELLES SIMULANT L'APOPTOSE

(30) Priority: 18.09.2001 US 323502 P
(43) Date of publication of application: 16.06.2004
(73) Proprietor: Vasogen Ireland Limited, Shannon, County Clare (IE)
(72) Inventor: BOLTON, Anthony, E., Bakeswell, Derbyshire DE45 1BJ (GB); MANDEL, Arkady, North York, Ontario M2R 3S7 (CA)
(74) Representative: Brown, David Leslie
(86) International application number: PCT/CA2002/001396
(87) International publication number: WO 2003/024422

(56) References cited:
- WO-A-02/24162
- WO-A-90/11781
- WO-A-99/13890
- US-A- 4 749 585
- US-A- 5 770 234
- US-A- 6 086 552
- FADOK V A ET AL: "A RECEPTOR FOR PHOSPHATIDYLSERINE-SPECIFIC CLEARANCE OF APOPTOTIC CELLS" NATURE, MACMILLAN JOURNALS LTD. LONDON, GB, vol. 405, no. 6782, 4 May 2000 (2000-05-04), pages 85-90, XP001120051 ISSN: 0028-0836
- FADOK V A ET AL: "MACROPHAGES THAT HAVE INGESTED APOPTOTIC CELLS IN VITRO INHIBIT PROINFLAMMATORY CYTOKINE PRODUCTION THROUGH AUTOCRINE/PARACRINE MECHANISMS INVOLVING TGF-BETA, PGE2, AND PAF" JOURNAL OF CLINICAL INVESTIGATION, NEW YORK, NY, US, vol. 101, no. 4, February 1998 (1998-02), pages 890-898, XP001120047 ISSN: 0021-9738

## Description

### FIELD OF THE INVENTION

This invention relates to therapeutic compositions and uses thereof in medical treatments and prophylaxis to lessen the effects of adverse medical conditions. More specifically, it relates to acceleration of recovery of a patient from the physical trauma of surgery and other wounding and injury conditions.

### BACKGROUND OF THE INVENTION

There is a continuing need to shorten the hospital stay of patients undergoing surgical procedures, which effectively means accelerating the rate of recovery of a patient from the trauma of surgery or other injuries. This applies both to patients undergoing pre-scheduled or elective surgery, and to patients undergoing surgery as a result of accidental injury or treatment of an unforeseen medical emergency. Both for the comfort and rapid recovery of the patient, and for the benefit of health care economics, it is desirable to be able to accelerate the rate of recovery of a patient from trauma after surgery or after suffering an accidental wound or injury.
It would also be desirable to be able to pre-condition a patient scheduled to undergo surgery, so that the patient would be better able to withstand the trauma associated with surgery, to lead to a more rapid recovery from trauma afterwards. It would also be advantageous to be able to precondition persons at risk of sustaining injury (battle troops, rescue personnel and the like) to enable them to recover more rapidly from such trauma.

WO90/11781 discloses methods for delivering therapeutic agents to wounds in liposomes which preferentially bind to the wounds are disclosed. Methods for delivering therapeutic agents in liposomes to wounds on the ocular surface of the eye are also disclosed.

US5770234 discloses a non-specific host defence cell augmentation technique for enhanced microorganism killing utilising any phagocytosable particle to prime macrophages for enhanced oxidative response and bacterial killing. The phagocytosable particles should be administered at the time of exposure to contagion, or one day prior to or up to 6-12 hours after exposure. Administration can be performed by any suitable means which will bring the particles quickly into contact with the blood stream where they will encounter phagocytes and cause priming of the patient's macrophages. The augmentation technique provides for non-specific cellular immunity from a wide range of contagion.

### SUMMARY OF THE INVENTION

The present invention is based upon the novel appreciation of the role played by the up-regulation of and-inflammatory cytokines and/or the down regulation of inflammatory cytokines in a patient's body, and by improved endothelial function, on the mammalian body's process of recovery from the trauma of surgery and other wounds. The natural process of apoptosis (programmed cell death) leads to the upregulation of and-inflammatory cytokines and the down-regulation of inflammatory cytokines in the mammalian body, as well as improvements in endothelial function. The present invention enables a process of accelerating the recovery of a patient from the trauma of wounds (surgical or accidental), and a process of pre-conditioning to accelerate the recovery from subsequently experienced such trauma, which mimics the apopotosis process of the mammalian body and takes advantage of the beneficial effects flowing from apoptosis in vivo, to effect such processes.

The present invention enables a process of treating a mammalian patient suffering from post-wounding trauma, or treating a mammalian patient about to or likely to suffer such trauma (by surgical treatment, or by suffering unanticipated accidental injuries, batde injuries or the like) to lessen the severity of or accelerate the recovery from such subsequently sustained trauma, comprising administering to the patient an effective immune system modifying amount of immune system-modifying entities, each comprising a body of a size similar to an apoptotic mammalian cell or apoptotic body, and having exposed on the surface phoapho-amino acid groups, the entities being capable of being taken up by cells of the patient's immune system with accompanying beneficial effects including inhibition of pro-inflammatory cytokines and/or promotion of anti-inflammatory cytokines.

### THE PREFERRED EMBODIMENTS

One category of such entities is natural biological vesicles presenting phosphatidylserine (PS) on an outer membrane surface. Such vesicles, upon administration to a mammalian patient, will mimic the apoptosis process with consequent down-regulation of pro-inflammatory cytokines and/or upregulation of anti-inflammatory cytokines. Immune cells can engulf the entities as the PS groups on the membranes thereof interact with the PS receptors in an *in vivo* process resembling apoptosis, with consequent down-regulation of pro-inflammatory cytokines and/or upregulation of anti-inflammatory cytokines. Natural biological vesicles presenting PS on an external membrane surface, include the following:
Exosomes, which are microvesicles exfoliated from cultured cells, and may also be produced in vivo, e.g. during maturation of reticulocytes (see Trams et. al., *Biochimica et Physica Acta,* 645 (1981) 63 - 70; and also Johnstone, *Biochem, Cell*. *Biol*., 70 (1982) 179 -190);
Prostasomes, which are vesicular extracellular organelles found in seminal plasma (see Rooney et. al., *J. Exp. Med*., 177, May 1993, 1409 - 1420);
Spontaneous or induced shed membrane vesicles, i.e. membrane vesicles shed from cells as a result of inducement using detergents such as lysophasphaddylcholine, or spontaneously (see Ferber et.al., *Biochimica* et *Biophysica Acta*, 595 (1980) 244-256; also Emerson et. al., *The Journal of Immunology*, 127(2), August 1981, 482-486);
Procoagulant bound to plasma membrane vesicles, i.e. thromboplastin-like activity associated with membrane vesicles, found for example in bronchoalveolar lavage fluid and derived from alveolar macrophages (see Lyberg et. al, *Eur. Respir. J.*, 3 (1990). 61 - 67);
Inside out red blood cell ghosts, which express PS on the outer surface, and sickle cell red blood cells which express PS on the surface as part of the pathology (see Schroit et. al, *Biol. Cell* 51 (1984) 227 - 238);
Erythrocytes with lost phospholipid asymmetry, i.e. erythrocytes with randomized, symmetric transbilayer distribution of phospholipids; these can be produced, for example, by elevating intracellular Ca⁺⁺ levels (see Pradham et al. *Molecular Membrane Biology* 11 (1994) 181 -188);
Activated platelets, platelets with pro-coagulant activity, which are associated with re-orientation of PS from the inner to the outer leaflet of the platelet membrane bilayer (see Bevers et. al., *Biochimica et Biophysica Acta*, 736; (1983) 57 - 66);
Platelet derived microparticles, which are membranous vesicles or microparticles shed from platelet membranes following platelet activation (see Gilbert et. al., *The Journal of Biological Chemistry*, 266 No.26, Sept. 16, 1991, 17261 - 17268).

Such natural biological vesicles can be used for administration to patients about to suffer trauma, e.g. patients about to undergo surgery or at high risk of suffering trauma from wounds as a result of imminent battle action, natural disaster etc., and will precondition the patient's body so as to accelerate the recovery from such trauma. They will also have the effect of accelerating the recovery of a patient from trauma when administered to an already traumatized patient.

Another category of such entities, the use of which in treatment of trauma and preconditioning against trauma constitutes another embodiment of the present invention, is biocompatible synthetic entities comprising:
a three-dimensional head portion of size in its largest dimension of from 50 nanometers ∞ 500 microns;
a plurality of tail portions bonded to each said head portion, the tail portions having:
   phospho-amino acid end groups capable of interaction with receptors on antigen-presenting cells,
   and chemical spacer groups of at least 3 linear carbon atoms, the spacer groups being bonded at cheir proximal ends to the respective head portion, and at their distal ends to the phosphare of the phospho-amino acid group.

The phospho-amino acid groups forming the end groups of the entities have the general formula: 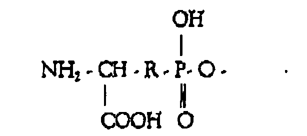 in which R represents C1 - C4 straight chain or branched alkylene, alkylene-oxy, alkylene-thio, alkylene-amine, phenyl, iodo-substituted phenyl, and 5-membered N-heterocyclic groups, with the proviso that they interact with appropriate receptors on antigen-presenting cells.
Preferred phospho-amino acid groups in the compositions are phospho-serine and phosphothreonine groups, with the most preferred being phosphoserine of formula:

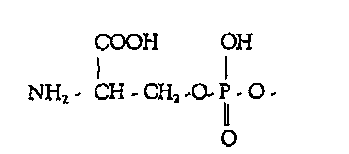

It is particularly preferred that the tail portions of the derivatized beads have the chemical formula: 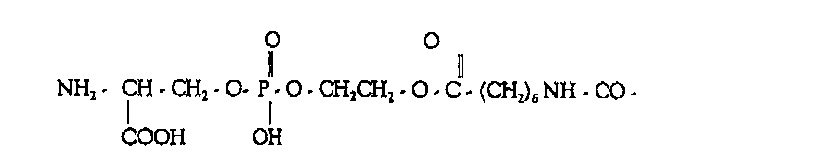 the amide end group being bonded to the head portion surface.

The term "beads" as used herein is intended to mean substantially any biocompatible body, solid, semisolid or hollow, shape-retaining and typically but not exclusively spheroidal, cylindrical, ellipsoidal including oblare and prolate spheroidal, serpentine, reniform, etc., and from about 50 nanometers to about 500 microns in diameter. They may be flexible or rigid. Preferred materials for their composition are polymethylmethacrylate, polacrylate, polymethacrylate, glass, polystyrene, polyethylene, polypropylene end the like, of a grade approved for administration to mammalian patients.

The phospho-amino acid end groups in the entities may be the distal end group of a phospholipid, the proximal end of which is attached to a body. These include particles, granules, microspheres or beads of biocompatible materials, natural or synthetic, such as polyethylene glycol, polyvinylprrolidone, polystyrene, etc., polysaccharides such as hydroxethyl starch, hydroxyethylcellulose, agarose and the like, as commonly used in the pharmaceutical industry. Some such suitable substances for derivatization to attach the PS and, in the case of agarose, with PS attached, are commercially available, e.g. from Polysciences, Inc. 400 Valley Road, Warrington, PA 18976, or from Sigma Aldrich Fine Chemicals. The beads may be solid or hollow, or filled with biocompatible material. They are modified as required so that they carry PS molecules on their surfaces.

Such phospho-amino acid carrying entities can be used for administration to patients about to suffer trauma involving wounds, e.g. patients about to undergo surgery or at high risk of suffering a wound as a result of imminent battle action, natural disaster etc., and will precondition the patient's body so as to accelerate the recovery from such subsequently encountered trauma. They will also have the effect of accelerating the recovery of a patient when administered to an already traumatized patient.

A further category of suitable entities is liposomes of the appropriate sizes referred to above, i.e., sizes resembling those of apoptotic mammalian cells or apoptotic bodies, and which have surface PS molecules. As a phospholipid, PS can form the membrane of a liposome, either as the sole constituent of the membrane or as a major or minor component thereof, with other phospholipids and/or membrane forming materials. Liposomes, or lipid vesicles, are sealed sacs, in the micron or sub-micron range, the walls of which consist of layers of suitable amphiphiles. They normally contain an aqueous medium.

Phospholipids are amphiphilic molecules (i.e. an amphiphiles), meaning that the compound comprises molecules having a polar water-soluble group attached to a water-insoluble hydrocarbon chain. The amphiphiles serving as the layers of the matrix have defined polar and apolar regions. The amphiphiles can include naturally occurring lipids such as PS, phosphatidylethanolamine, phosphatidylinositol, phosphaddylcholine, cholesterol, ceramides and sphingomyelin, used alone or in admixture with one another. They can be synthetic compounds such as polyoxyethylene alkylethers, polyoxyethylene alkylesters and saccharosediesters.

The use, not only of those liposomes having PS as a membrane constituent, but also liposomes having non-PS membrane substituent but which carry on their external surface molecules of PS, e.g., chemically attached by chemical modification of the liposome surface is contemplated making the PS available for subsequent interaction with components of the patient recipient's immune system.

Preferred are liposomes constituted to the extent of 50% 100% by weight of phosphatidylserine (PS), the balance being phosphatidylcholine (PC) or other such biologically acceptable phospholipid(s). More preferred are liposomes constituted by PS to the extent of 65% - 90% by weight. They are prepared from mixtures of the appropriate amounts of phospholipids as starting materials, by known methods,

Methods of preparing liposomes of the appropriate size are known in the art and do not form part of this invention. Reference may be made to various textbooks and literature articles on the subject, for example the review article "Liposomes as Pharmaceutical Dosage Forms" by Yechezkel Batenholz and Daan J. A. Chromeline, and literature cited therein, for example New, R. C., "Liposomes; A Practical Approach," IRL Press at Oxford University Press, Oxford, England (1990), and Nassander, U. K., et al., In: "Biodegradable Polymers as Drug Delivery Systems" (M. Chasin and R. Langer, eds.) Marcel Dekker Inc., New York 1990, pages 261-338.

Such PS-carring liposomes can be used for administration to patients about to suffer trauma involving wounds, e.g. patients about to undergo surgery or at high risk of suffering a wound as a result of imminent battle action, natural disaster etc., and will precondition the patient's body so as to accelerate the recovery of such subsequently encountered trauma. They will also have the effect of accelerating the recovery of a patient when administered to an already traumatized patient.

A further category of entities suitable for use is apoptotic cells and apoptotic bodies themselves. "Apoptotic cells" and "apoptotic bodies," as the terms are used herein, means cells and cell bodies which exhibit one or more of the following apoptosis-characterizing features: surface exposure of phosphatidylserine, as detected by standard, accepted methods of detection such as Annexin V staining; alterations in mitochondrial membrane permeability measured by standard, accepted methods (e.g. Salvioli, S., Ardizzoni, A., Franceschi, C. Cossarizza, A. (1997) "JC-1, but not DiOO6(3) or Rhodamine 123, is a Reliable Fluorescent Probe to assess Delta Psi Changes in Intact Cells: Implications for Studies on Mitochondrial Functionality during Apoptosis," *FEBS Letters* 411; 77-82]; evidence of DNA fragmentation such as the appearance of DNA laddering on agarose gel electrophoresis following extraction of DNA from the cells [Teiger, E., Dam, T.V., Richard, L., Wisnewsky, C., Tea, B.S., Gaboury, L., Tremblay, J., Schwartz, K. and Hamet, P. (1996) "Apoptosis in Pressure Overload-induced Heart Hypertrophy in the Rat," *Journal of Clinical Investigation* 97; 2891-2897], or by in situ labeling (see Gavrieli et al., 1992, referenced above).

The apoptotic cells and/or apoptodc bodies suitable for use are prepared *ex vivo* from mammalian cells that are compatible with those of the mammalian patient to whom they are to be administered. They can be prepared from substantially any type of mammalian cell including cultured cell lines. Preferably they are prepared from a cell type derived from the mammalian patient's own body or from an established cell line. More preferably they are prepared from white blood cells of blood compatible with that of the mammalian patient, more preferably from the patient's own white blood cell and even more preferably from the patient's own T lymphocytes. Even more preferably they are prepared from an established cell line. The apoptotic cells and/or apoptotic bodies are prepared extracorporeally prior to administration to the patient. Thus, in one embodiment, an aliquot of the patient's blood may be withdrawn, e.g. by venipuncture, and at least a portion of the white cells thereof subjected extracorporeally to apoptosis inducing conditions.

A variety of methods of inducing apoptosis in mammalian cells, so as to create apoptotic cells and apoptotic bodies, are known in the art and essentially any of these can be adopted in preparing apoptotic bodies for use in the present invention. One such method is the subjection of the cells to ionizing radiation (γ-rays, x-rays, etc.) and/or non-ionizing electromagnetic radiation including ultraviolet light. Apoptosis can be induced by subjecting cells to ultrasound.

Another method is the treatment of the cells with drugs such as non-specific protein kinase inhibitors as exemplified by staurosporine (see Bombeli, Karsan, Tait and Hirlan, (1997) "Apoptotic Vascular Endothelial Cells Become Procoagulant", Blood, Vol. 89:2429-2442). Also, certain chemotherapeutic agents used for the treatment of malignant tumours induce apoptosis, for example adriamycin, as can statin drugs (3-hydroxy-3methylglutaryl coenzyme A reductase inhibitors) [Guijarro C, Blanco-Colio LM, Ortego M, Alonso C, Ortiz A, Plaza JJ, Diaz C, Hernandez G, Edigo J (1998), "3-hydroxy-3methylglutaryl coenzyme A reductase and isoprenylation inhibitors induce apoptosis of vascular smooth muscle in culture," *Circulation Research* 83: 490-500] and colcicine [Suzuki Y (1998) ", "Cell death, phagocytosis and neurogenesis in mouse olfactory epithelium and vomeronasal organ after colcicine treatment," Annals *of the New York Academy of Sciences* 855: 252-254]. The use of ligands for death receptors on cells, such as Fas-ligand, will be apparent for inducing apoptosis from the discussion of apoptosis above.

Yet another method is the application of oxidative stress to cells extracorporeally (see for example Buttke and Sandatrom (1994) "Oxidative Stress as a Mediator of Apoptosis," Immunology Today, Vol.15:7-10). This can be achieved by treating the cells, in suspension, with chemical oxidizing agents such as hydrogen peroxide, other peroxides and hydroperoxides, ozone, permanganates, periodates, and the like. Biologically acceptable such oxidizing agents are preferably used, so as to reduce potential problems associated with residues and contaminations of the apoptotic cells and apoptotic bodies so formed.

Any suitable method of producing apoptotic cells and apoptotic bodies, for use herein, can be used.

Methods for the detection and quantitation of apoptosis can be used to determine the presence and level of apoptosis in the preparation to be administered to the patient. At least one of the methods from those described in the introduction above should be used to confirm the level of apoptosis achieved prior to administration. They are suitably purified prior to use, by methods known in the art, such as differential centrifugation.

One preferred process of preparing apoptotic cells and apoptotic bodies for use involves the culture of cells from the patient, or a compatible mammalian cell line. The cultured cells may then be treated to induce apoptosis and to create apoptotic cells and/or apoptotic bodies therein. The cells, suspended in the patient's plasma or another suitable suspension medium, such as saline or a balanced mammalian cell culture medium, can then be administered as indicated below. The numbers of apoptotic cells and/or bodies can be determined by published methods available in the scientific literature on die subject including the above-reference articles. The numbers of such apoptotic cells and/or apoptotic bodies required for administration to the patient to obtain the required clinical benefit will vary depending on the source of cells, the patient's condition, the age and weight of the patient and other relevant factors which are readily determinable by the attending clinician.

The successful application of the process may be manifested in several ways, individually or collectively. The patient may manifest accelerated rate of wound healing, and/or more rapid decline of elevared body temperatures resulting from inflammatory cytokine action and fever as a result of wounding. In addition or in the alternative, the patient may evidence a more rapid recovery of joint mobility, e.g. following orthopedic surgery to replce or to repair a defective body joint (knee, hip, shoulder, etc). A greater survival rate of seriously injured patients is to be anticipated as a result. Furthermore, the duration of the hospital stay for the patient can be significantly reduced.

Another common manifestation of patients obliged to spend long periods in bed as a result of trauma from injury is the development of medical ulcers (decubitus or pressure ulcers). The processes are indicated for acceleration of the healing of such ulcers, and indeed for treating and accelerating the healing of mammalian ulcers in general, and thereby further contributing to the shortening of the duration of a patient's hospital stay,

The sizes of the immune modifying entities used are such that they will be taken up by cells of the patient's immune system in an apoptosis-mimicking fashion. In general, whatever type of entity is chosen, this means a size from about 50 nanometers to about 500 microns, more preferably from about 50 nanometers to about: 500 nanometers.

The entities used in the process may be administered to the patient by any suitable means which brings them into operative contact with active ingredients of the patient's immune system. Preferably, the entities are constituted into a liquid suspension in a biocompatible liquid such as physiological saline and administered to the patient intra-arterially, intravenously or most preferably intramuscularly or subcutaneously.

A preferred manner of administering the entities to the patient is as a course of injections, administered daily, several times per week, weekly or monthly to the patient, over a period ranging from a week to several months. The frequency and duration of the course of the administration is likely to vary widely from patient to patient, and according to the severity of the trauma being treated or against which the patient is to be preconditioned. Its design and optimization is well within the skill of the attending physician.

The quantities of entities to be administered will vary quite widely depending on the severity of the trauma it is intended to treat or against which it is intended to precondition, and on the identity and characteristics of the patient. It is important that the effective amount of entities is non-toxic to the patient, and is not so large as to overwhelm the immune system.

When using intra-arterial, intravenous, subcutaneous or intramuscular administration of a liquid suspension of entities, it is preferred to administer, for each dose, from about 0.1-50 ml of liquid, containing an amount of entities generally equivalent to 1.0% - 1000% of the number of cells normally found in an equivalent volume of whole blood or the number of apoptotic bodies that can be generated from them. Generally, the number of synthetic entities administered per delivery to a human patient is suitably in the range from about 500 to about 20 x 10⁹, preferably 10,000 to about 2 x 10⁹, as indicated by pre-clinical studies. Since animal models may not be truly representative of required numbers on a simple multiple of body weight, in an immune system modifying scenario, useful human dosage amounts may also be found in the 500 - 20,000,000 range.

Since the synthetic entities are acting, as immune system modifiers, in the nature of a vaccine, the number of such bodies administered to an injection site for each administration is a more meaningful quantitation than the number or weight of synthetic entities per unit of patient body weight. For the same reason, effective amounts or numbers of synthetic entities for small animal use may not directly translate into effective amounts for larger mammals on a weight ratio basis.

The invention is further described, for illustrative purposes, in the following specific example.

### EXAMPLE

The invention can be demonstrated by experiments on laboratory rats, pretreating them with a course of injections of phosphatidylserine liposomes, surgically inserting temperature and heartbeat measuring probes into the pre-treated animals, and measuring their body temperature and other vital signs using the probes, as a measure of their recovery from the surgical major laparotomy required for insertion. The results are predictive of the effects on other mammals, including humans.
A total of 30 seven week old laboratory bred rats is separated into two of 15 animals each. Each animal of the test group A is administered, on day 1, day 2 and day 14, an intragluteal injection of 75% phosphatidylserine - 25% phosphatidylcholine liposomes of size 100± 20 nanometers, suspended in PBS, of volume 150 µL, each injection comprising 1,800,000 liposomes. Each animal of the control group B is similarly administered 50 µL of PBS containing no liposomes, on days 1, 2 and 14.
Four days after the completion of the injections, the animals are anaesthetized, and a telemetry probe is inserted surgically into the femoral artery of each animal. The telemetry probe (DATAQUEST LABPRO, from Data Sciences International) is a commercially available probe equipped with a radio transmitter, to permit heartbeat, systolic blood pressure, diastolic blood pressure and other signals to be received without further handling of the animals. An additional probe is surgically inserted into the peritoneal cavity of each animal, to measure body temperature.
Continuous daily recordings of body temperature, blood pressure and heart rate are made from each animal, for 10 days following the surgery. The group A rest animals show a noticeably faster recovery of normal body temperature than the control group B, demonstrating a faster rate of wound healing and recovery from surgery in the test group.

## Claims

1. Use of liposomes which comprise from 50% to 100% phosphatidylserine (PS) by weight having PS exposed on a surface thereof or synthetic beads having PS exposed on a surface thereof the liposomes or synthetic beads being of a size similar to an apoptotic mammalian cell or apoptotic body, in the preparation of a medicament for treating a mammalian patient suffering from post-wounding trauma to lessen the severity of or accelerate the recovery from such trauma, or of a mammalian patient about to or likely to suffer trauma of wounding or undergoing surgery to lessen the severity of or accelerate the recovery from such subsequently sustained trauma, with accompanying inhibition of pro-inflammatory cytokines and/or promotion of anti-inflammatory cytokines on the liposomes or beads being taken up by cells of the patient's immune system.

2. Use according to claim 1 wherein PS-carrying synthetic beads are used.

3. Use according to claim 1 wherein PS-carrying liposomes are used.

4. Use according to any one of the preceding claims wherein the liposomes or synthetic beads have a diameter of from 50 nanometers to 500 micrometers.

5. Use according to any one of claims 1 to 4 wherein the medicament comprises a unit dose of from about 500 to 20 x 10⁹ of the liposomes or synthetic beads.

6. Use according to claim 5 wherein the medicament comprises a unit dose of from about 500 to 2,000,000 of the liposomes or synthetic beads.

## Patentansprüche

1. Verwendung von von 50 Gew.-% bis 100 Gew.-% Phosphatidylserin (PS) enthaltenden Liposomen, wobei das PS an deren Oberfläche exponiert ist, oder synthetischen Kügelchen, wobei das PS an deren Oberfläche exponiert ist, wobei die Liposomen oder synthetischen Kügelchen von ähnlicher Größe wie eine apoptotische Säugerzelle oder ein apoptotisches Körperchen sind, zur Herstellung eines Arzneimittels zur Behandlung eines an einem Trauma nach Verletzung leidenden Säugerpatienten zur Verminderung der Schwere eines oder der Beschleunigung der Erholung von einem solchen Trauma(s) oder eines Säugerpatienten, der im Begriff oder für den es wahrscheinlich ist, das Trauma einer Verletzung zu erleiden oder sich einer Operation zu unterziehen, zur Verminderung der Schwere eines oder zur Beschleunigung der Erholung von einem solchen nachfolgend erlittenen Trauma(s) mit begleitender Hemmung entzündungsbegünstigender Cytokine und/oder Unterstützung antientzündlicher Cytokine an den von Zellen des Immunsystems des Patienten aufgenommenen Liposomen oder Kügelchen.

2. Verwendung nach Anspruch 1, worin PS-tragende synthetische Kügelchen verwendet werden.

3. Verwendung nach Anspruch 1, worin PS-tragende Liposomen verwendet werden.

4. Verwendung nach einem der vorangehenden Ansprüche, worin die Liposomen oder synthetischen Kügelchen einen Durchmesser von 50 Nanometer bis 500 Mikrometer haben.

5. Verwendung nach einem der Ansprüche 1 bis 4, worin das Arzneimittel eine Dosiseinheit von etwa 500 bis 20 x 10⁹ der Liposomen oder synthetischen Kügelchen enthält.

6. Verwendung nach Anspruch 5, worin das Arzneimittel eine Dosiseinheit von etwa 500 bis 2 000 000 der Liposomen oder synthetischen Kügelchen enthält.

## Revendications

1. Utilisation de liposomes comprenant entre 50 % et 100 % en poids de phosphatidylsérine (PS) exposée sur une surface de ceux-ci ou de perles synthétiques possédant de la PS exposée sur une surface de celles-ci, les liposomes ou perles synthétiques étant d'une taille similaire à celle d'une cellule de mammifère apoptotique ou de corps apoptotique, dans la préparation d'un médicament destiné à traiter un patient mammifère souffrant d'un traumatisme post-lésionnel afin d'atténuer la sévérité de ce traumatisme ou d'en accélérer la guérison, ou un patient mammifère sur le point de subir un traumatisme lésionnel, ou susceptible de le subir, ou de subir une intervention chirurgicale, afin d'atténuer la sévérité de ce traumatisme ultérieurement subi, ou d'en accélérer la guérison, avec inhibition concomitante des cytokines pro-inflammatoires et/ou promotion des cytokines anti-inflammatoires envers les liposomes ou perles qui sont absorbés par les cellules du système immunitaire du patient.

2. Utilisation selon la revendication 1, dans laquelle des perles synthétiques portant de la PS sont utilisées.

3. Utilisation selon la revendication 1, dans laquelle des liposomes portant de la PS sont utilisés.

4. Utilisation selon l'une quelconque des revendications précédentes, dans laquelle les liposomes ou perles synthétiques présentent un diamètre compris entre 50 nanomètres et 500 micromètres.

5. Utilisation selon l'une quelconque des revendications 1 à 4, dans laquelle le médicament comprend une dose unitaire comprise entre approximativement 500 et 20 x 10⁹ de liposomes ou perles synthétiques.

6. Utilisation selon la revendication 5, dans laquelle le médicament comprend une dose unitaire comprise entre approximativement 500 et 2 000 000 de liposomes ou perles synthétiques.
